(19) Europäisches Patentamt
European Patent Office
Office européen des brevets

(11) **EP 2 292 144 A1**

(12) **EUROPEAN PATENT APPLICATION**

(43) Date of publication:
**09.03.2011 Bulletin 2011/10**

(51) Int Cl.:
*A61B 5/12* (2006.01)   *H01R 25/00* (2006.01)

(21) Application number: **09169425.7**

(22) Date of filing: **03.09.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
• **National Digital Research Centre**
**8 Dublin (IE)**
• **Cleartone Technologies Limited**
**Dartry, Dublin 6 (IE)**
• **University College Dublin, National University of Ireland Dublin**
**Dublin 4 (IE)**

(72) Inventors:
• **Ganter, Declan B.**
**6, Dublin (IE)**

• **Graham, Fintan J.**
**Co. Wicklow (IE)**
• **Ganter, Martin C.**
**Co. Dublin (IE)**
• **Rickard, Scott**
**Co. Dublin (IE)**
• **Barry, Dan**
**22, Dublin (IE)**
• **Jennings, Kevin**
**5, Dublin (IE)**
• **Kozlov, Denis**
**9, Dublin (IE)**

(74) Representative: **Reedy, Orlaith et al**
**Tomkins & Co**
**5 Dartmouth Road**
**Dublin 6 (IE)**

(54) **An auditory test and compensation method**

(57)    The present invention provides an auditory test and compensation method for an audio system comprising an audio device coupled to an audio output means and a listener of the audio device. The method comprises the steps of: delivering a series of audio stimuli through the audio output means; capturing a listener's response to the audibility of the stimuli; calculating a compensation print from the frequency response; deriving a filter from the calculated compensation print with respect to the frequencies associated with the frequency response and applying the filter to an audio signal of the audio system.

Deliver a series of audio stimuli through the audio output and capture the listener's response

Process the captured response to generate a frequency response of the audio system

Process the frequency response to generate a system compensation filter

Apply the compensation filter to the audio signal

Figure 2

EP 2 292 144 A1

**Description**

**Field of the Invention**

The present invention relates to the field of audio signal processing. More particularly, the invention relates to personal auditory compensation filtering for the purposes of audio system optimisation.

**Background to the Invention**

Audiometric tests are commonly performed on an individual experiencing hearing difficulties. These typically involve a healthcare professional performing a hearing test on the individual using a calibrated system comprising an analog headset and an analog tone generator, with the generator adapted to generate pure tones at a plurality of test frequencies and at different volume levels. When the system requires calibration, both the headset and the tone generator must be calibrated together.

An improvement in such audiometric tests is disclosed in EP Patent Publication No. 2 005 792, which describes a calibrated digital audiometric testing system for generating a user hearing profile. This system has the advantage that it requires only the headset to be calibrated, rather than the entire system. Furthermore, it discloses the programming of an audio device with the hearing profile.

There exists a need to provide an auditory test method for use by an individual which can be performed in both calibrated and uncalibrated test environments and which enables a user to optimise their listening experience on a particular audio device.

**Summary of the Invention**

The present invention provides an auditory test method for an audio system comprising an audio device coupled to an audio output means and a listener of the audio device, the method comprising the steps of:

> delivering a series of audio stimuli through the audio output means;
> capturing a listener's response to the audibility of the stimuli; and
> generating a frequency response for the audio system based on the captured data.

The method may further comprise the steps of:

> a) generating a tone of a predefined frequency and amplitude;
> b) repeatedly reducing the amplitude of the generated tone until the captured response indicates an amplitude value which is not audible to the listener;
> c) storing the relative amplitude value as the threshold amplitude value of the listener for the frequency;
> d) repeating steps a) to c) for a plurality of different frequencies within a predefined frequency range; and
> e) generating the frequency response for the audio system from the stored threshold amplitude values for the frequencies.

The method may further comprise the steps of:

> generating a band limited noise burst encapsulating a plurality of frequencies greater than the predefined frequency range;
> determining from the captured response the threshold frequency value of the listener for the band limited noise burst; and
> generating the frequency response for the audio system based on the stored threshold amplitude values and the threshold frequency value of the band limited noise burst.

Preferably, the predefined frequency range is a range between 20Hz and 15Khz, and the band limited noise burst encapsulates frequencies between 15KHz and 20kHz.

Desirably, the frequencies are derived from the closest critical band centres to the test frequencies utilised in the ISO 226 standard.

The method may further comprise the steps of:

> generating a first chirp signal containing the threshold amplitude values for the frequencies;
> determining from the captured response whether the chirp signal is audible to the listener;

generating a second chirp signal containing amplitude values less than the threshold amplitude values for the frequencies;

determining from the captured response whether the second chirp signal is audible to the listener; and

indicating that the generated frequency response for the audio system is correct if all of the frequencies in the first chirp signal are audible and the second chirp signal is not audible to the listener.

The method may further comprise the step of detecting whether the listener has a hearing impairment based on the value of the generated frequency response.

The audio output means may comprise one or more of: speakers and headphones.

Suitable, the audio output means comprises headphones, and the method further comprises the step of delivering the series of audio stimuli to the left and right headphones over separate time periods.

The method may further comprise the step of uploading the generated frequency response to a remote server.

The present invention also provides a compensation method for an audio system associated with a frequency response which has been previously generated by the performance of an auditory test on one or more of the system components, the system comprising an audio device coupled to an audio output means and a listener of the audio device; the method comprising:

a) calculating a compensation print from the frequency response;

b) deriving a filter from the calculated compensation print with respect to the frequencies associated with the frequency response;

c) applying the filter to an audio signal of the audio system.

The step of calculating the compensation print may comprise:

performing a filter transformation which maps the frequency response to the ideal system response; and normalising the resultant vector.

The step of deriving the filter may further comprise the steps of:

mapping the frequency points associated with the frequency response to discrete fourier bins; and interpolating the calculated compensation print values with respect to the fourier bins indices so as to provide a compensation filter kernel having a length corresponding to the fourier transform length of each audio frame.

The step of applying the filter to the audio signal may comprise the step of: multiplying the filter kernel by the instantaneous short term fourier magnitude spectrum of each audio frame.

The method may further comprise the step of providing for the adjustment in real time of the magnitude of the filter applied to the audio signal.

The present invention also provides an auditory test and compensation method for an audio system comprising an audio device coupled to an audio output means and a listener of the audio device comprising:

generating a frequency response for the audio system; and

compensating for the frequency response of the audio system.

The present invention also provides an audio system comprising:

an audio device; and

an audio output means coupled to the audio device; and

a capturing means for capturing a listener's response to a series of audio stimuli delivered through the audio output means; and

a processor adapted to perform the auditory test and compensation method.

The processor may be provided in the audio device.

The processor may be provided in the audio output means.

The processor may be programmed by the downloading of a program from a remote source.

The present invention also provides a system comprising:

an audio system comprising an audio device coupled to an audio output means; and

a remote source for storing audio content for downloading to the audio device;

wherein the audio device is adapted to perform the auditory test method and upload the generated frequency response to the remote source; and the remote source is adapted to perform the compensation method on the frequency response and download the filtered audio signal to the audio device.

The present invention also provides an in-ear device programmed with a frequency response generated in accordance with the steps of the auditory test method and adapted to compensate for the frequency response in accordance with the steps of the compensation method.

The device may be a hearing aid.

## Brief Description of the Drawings

Figure 1 illustrates a block diagram of the main components of an audio system on which the auditory test and compensation method of the present invention is performed;

Figure 2 illustrates the main steps of the auditory test and compensation method;

Figure 3 illustrates the substeps of steps 1 and 2 of Figure 2;

Figure 4 details the substeps of steps 1 to 3 of Figure 3;

Figure 5 details the substeps of the process of steps 3 and 4 of Figure 2;

Figure 6 details the relationship between input and output frames for $\alpha=1$; and

Figure 7 details the real-time output buffer scheme using a 75% overlap.

## Detailed Description of the Drawings

The present invention discloses a system wide auditory test method adapted for use by an individual. It also discloses a method of compensating the audio output from the system based on the test results so as to enhance or optimise the listener's auditory experience.

The term 'system' is used to mean the encapsulated end to end listening chain including: one or more audio reproduction devices, the acoustic transducers, such as headphones and speakers, and the listener's human auditory system.

The invention will now be described in accordance with one embodiment, as shown in the figures. It should be understood that while this embodiment describes the invention when the system includes a single audio device, it can equally well be applied to a system incorporating a plurality of audio devices.

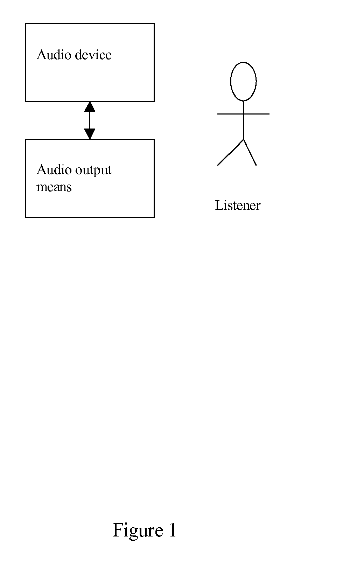

Figure 1 illustrates a block diagram of the main components of an audio system on which the auditory test and compensation method of the present invention is performed. It comprises an audio device, an audio output means, such as headphones and/or speakers, and a listener.

In order to carry out the invention, the testing and compensation program which, when executed, performs the auditory test and compensation method must first be installed on the audio system. The installation process is dependent on the type of audio device, and on which component of the audio system the compensation is to be performed, further details of which are discussed later. The listener is then provided with a remote button, and instructed to depress the button in response to hearing audio stimuli of known characteristics delivered through the headphones or through the speakers. The auditory test may then be started.

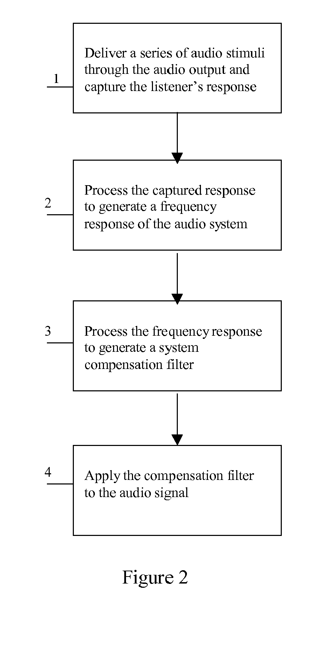

Figure 2 illustrates the main steps of the auditory test and compensation method of the present invention. In step 1, the series of audio stimuli is delivered to the audio output means, and the listener's response to the audibility of the stimuli is captured. In the preferred embodiment, the stimuli include pure tone, chirp signals and band limited noise bursts, but it will be appreciated that the type of stimuli can be selected depending on the level of accuracy desired for the compensation calculation. In step 2, the captured data is processed in order to generate a frequency response of the entire system. This will be referred to from hereon in as a "system print". In step 3, the system print is processed in order to generate a system compensation filter or profile which is applied to the audio of the system. The purpose of the compensation filter is to optimise the listener's auditory response to audio emitted from the audio device when located within the system. The system compensation filter is therefore inserted in the audio processing chain such that all audio content from the system is subjected to compensation. The actual process of applying the compensation filter to the audio may be performed in a number of ways, such as for example within the audio device, in the headphones or by means of an audio delivery application (step 4). This will be discussed in further detail later. It should be appreciated therefore that this system print is listener specific and is only valid for the specific system on which it was acquired.

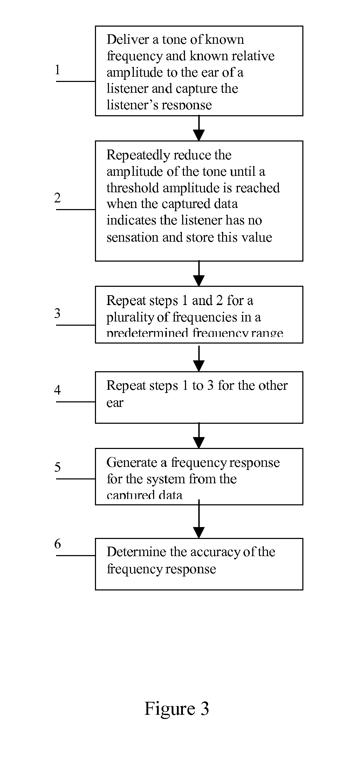

Figure 3 illustrates the various sub-steps in performing steps 1 and 2 of the method of Figure 2. This involves the delivery of a series of pure tones of known frequency and of known relative amplitude to the listener to perform the auditory test process on the listener. In step 1, a generated tone is output to one ear of the listener at a known relative amplitude at a particular frequency point. The listener's response is then captured, by the listener pressing the remote response button in order to indicate auditory sensation of the tone. The tone is then successively reduced in amplitude until a threshold is reached for which the listener indicates no sensation, by omitting to press the response button. In step 2, the relative amplitude value of this output tone is stored. In step 3, the process of steps 1 and 2 is repeated for a number of individual frequencies between a lower limit of 20 Hz and an upper limit of 20 KHz. These frequencies are chosen so as to accommodate enhanced hi-fidelity listening, as they relate generally to the bandwidth of human auditory sensation. In step 4, the process of steps 1 to 3 is repeated for the other ear. In step 5, the system print is then generated from processing the captured data. Finally, in step 6, the accuracy of the generated system print is determined.

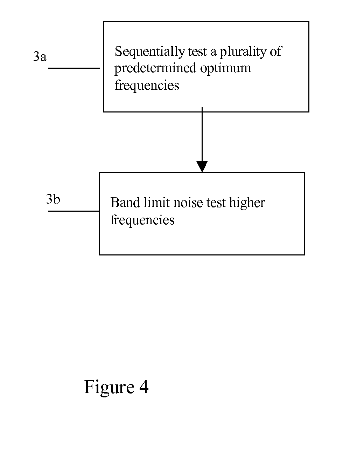

The process of steps 1 to 3 of Figure 3 comprises two substeps, namely the sequential testing of a plurality of predetermined optimum frequencies (step 3a) followed by band limited noise testing (step 3b), as shown in Figure 4 and described in more detail below.

With regard to step 3a, it will be appreciated that is not realistically feasible to test a large number of frequency points across the human auditory spectrum, due to the length of time it would take to process the data. In addition, listener fatigue could corrupt the response. Rather than using arbitrarily spaced or octave spaced frequency test points, an optimal subset of frequency points is selected such that there are just enough to interpolate a smooth frequency response. The specific test frequency points used for the sequential testing are derived from the centre frequencies of the auditory critical bands which fall closest to those frequency points used in the ISO 226 standard, which presents statistically ideal hearing sensitivity thresholds for a normally able listener. Previous research into human auditory perception has derived 24 critical bands within the human auditory system, which refer to specific locally grouped regions of sensitivity on the basilar membrane within the cochlea. This is due to the fact that it has been shown that within any given critical band, a minimal audible threshold shift is experienced in the presence of an acoustic stimulus (a phenomenon known as critical band masking). As such, frequency components with magnitudes below the newly shifted threshold will be imperceptible. By using this information, real discrete threshold data for each frequency point is provided, which can then be referenced during the compensation process. In the preferred embodiment of the invention, a subset of these bands is used for efficiency purposes. Table 1 below shows the selected specific test frequency points in italics.

| Bark Band No. | Centre Frequency (Hz) | Closest Test Frequency Points in ISO 226 (Hz) |
|---|---|---|
| 1 | 50 | *50* |
| 2 | 150 | *160* |
| 3 | 250 | 250 |
| 4 | 350 | *315* |
| 5 | 450 | 400 |
| 6 | 570 | *500* |
| 7 | 700 | 630 |
| 8 | 840 | 800 |
| 9 | 1000 | *1000* |
| 10 | 1170 | 1250 |
| 11 | 1370 | - |
| 12 | 1600 | 1600 |
| 13 | 1850 | - |
| 14 | 2150 | *2000* |
| 15 | 2500 | 2500 |
| 16 | 2900 | *3150* |
| 17 | 3400 | - |
| 18 | 4000 | *4000* |
| 19 | 4800 | 5000 |
| 20 | 5800 | *6300* |

(continued)

| Bark Band No. | Centre Frequency (Hz) | Closest Test Frequency Points in ISO 226 (Hz) |
|---|---|---|
| 21 | 7000 | - |
| 22 | 8500 | *8000* |
| 23 | 10500 | *10000* |
| 24 | 13500 | *12500* |
| 25 | N/A | *15000* (extrapolated using ISO 226 curve) |

It will be appreciated that in an alternative embodiment of the invention, one frequency point could be tested per critical band.

The band limited noise testing of step 3b takes account of the fact that it is common for large percentages of the population to experience age related high frequency desensitisation, that is to experience no sensation beyond a certain frequency. It will be appreciated that this threshold frequency will differ from listener to listener. However, it is not feasible to establish this threshold for each listener. In accordance with the present invention therefore, an approximate sensitivity threshold for all frequencies above 15KHz is established for the listener as a grouping. In the described embodiment of the invention, this is achieved by the delivery of a 2Hz modulated band limited noise burst to the listener.

As mentioned above, step 6 of the process of Figure 3 determines the accuracy of the data making up the system print. This is achieved by reproducing to the listener a chirp signal in which the instantaneous amplitude is frequency dependent. Specifically, the amplitude at any given frequency in the chirp signal is that of the sensitivity threshold derived during step 2 of the testing process of Figure 3. The chirp amplitude tracks the threshold curve derived through interpolation of the selected frequency points. This chirp is generated as follows:

$$S_{(n)} \times \sin(2\pi f_{(n)}t + \Phi) \qquad 20Hz \leq f \leq 20KHz$$

where $S(n)$ is an interpolated representation of the sensitivity threshold amplitudes derived from the testing, and $f(n)$ is a vector of instantaneous frequency values at time t with phase $\Phi$.

This creates a chirp signal containing all frequencies from 20Hz to 20Khz at continuously varying amplitudes dependent on the sensitivity thresholds derived.

In step 6, this chirp is reproduced to the listener at the sensitivity threshold, and the listener is requested to indicate sensation by depressing the remote button. The entire chirp is then reproduced to the listener at decreased amplitude (relative to values in $S$). If the system print is accurate, it will be appreciated that all of the frequencies for the chirp corresponding to the sensitivity thresholds should be audible to the listener, while no sensation should be experienced for the chirp at the decreased amplitude. Therefore, if full or partial sensation is reported by the listener, or if not all of the frequencies are audible to the listener for the chirp at the sensitivity thresholds, an error has been detected in the testing stage. If this occurs, the testing process described with reference to steps 1 to 4 of Figure 3 should be partially or fully repeated. Otherwise, the next stage in the process should be performed, namely the generation of a compensation filter or profile for the system print.

The above described steps result in the derivation of a system print for any listener/system combination. In this regard, it should be noted that the data provided by ISO 226 was derived using an ideal audio reproduction system. This means that all measures were taken to ensure that the reproduction system itself was calibrated to have a uniform frequency response, and that the data corresponded specifically to human hearing threshold measurements alone. However, in contrast, the method of the present invention does not assume a uniform frequency response in the audio system, nor are hearing thresholds measured. Instead, the combined system response, $S(n)$, was measured, which encapsulates all aspects of the listening chain including the system and the listener. It consists of a set of discrete frequency values measured in Hertz (Hz) and a related set of threshold sensitivity values measured in decibels (dB).

Once the system print is generated, the next step in the process is to calculate a system compensation filter (step 3 of Figure 2). The system compensation filter is designed to approximate an optimised listening condition for the listener/system combination, by processing all audio content such that the perceived frequency response for the listener on a non calibrated system is approximate to that of a 'normal' listener on a system with a uniform frequency response, i.e. the ideal listening condition.

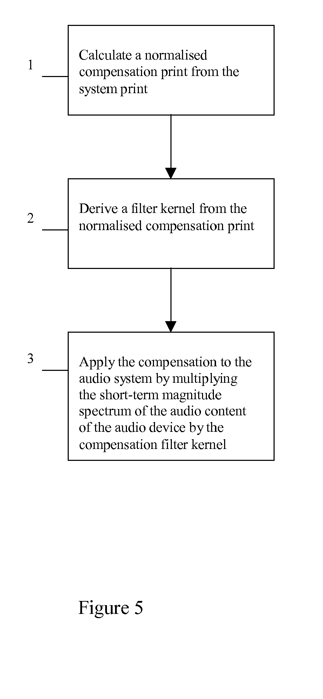

Figure 5 details the substeps of the process of steps 3 and 4 of Figure 2, namely that of generating a system compensation filter from the system print output from the test step, which is then applied to the audio system. It requires the calculation of frequency dependent offset gains which approximate a known 'ideal' listening condition as in ISO 226. In step 1, a normalised compensation print is calculated from the system print. In step 2, a 2048 point linearly spaced filter kernel is derived from the normalised compensation print with respect to the frequency points used during the test process. In step 3, the compensation is applied to the audio system by the short-term magnitude spectrum of the audio content of the audio device of the system being multiplied by the compensation filter kernel. These steps are described in more detail below.

The compensation print $C(n)$ of step 1 is calculated by means of a filter transformation which maps the system print, $S(n)$, to the target (ideal) system response, $T(n)$, both measured in dB. The target system response is a subset of corresponding data from the normal hearing thresholds described in ISO 226. The transformation is as follows:

$$C_{(n)} = T_{(n)} - S_{(n)} \qquad 1 \leq n \leq N$$

where $(n)$ is a frequency index and $N$ is the number of frequency points comprising the system print. An additional vector of frequency values, F(n), specifies the discrete frequency points at which $S(n)$, $C(n)$ and $T(n)$ are taken.

As mentioned previously, the generated system print encapsulates all aspects of the listening chain including the system and the listener. Accordingly, it is important to note that in calculating $C(n)$, a listener's hearing deficiencies are not specifically compensated, nor are system non linearities compensated for. Rather, the listener/system combination is compensated. Therefore, if any part of the end to end listening chain is modified, the compensation is no longer valid, and a new system print will have to be derived and its necessary compensation calculated.

Given that $T(n)$ is actually measured in dB SPL and $S(n)$ is not, there may be an arbitrary shift in the resultant $C(n)$ vector. Assuming the system has linear dynamic transfer characteristic, this shift will simply correspond to a constant gain factor. In order to avoid unnecessary application of broadband gain (and thus wasting dynamic range), the compensation print, $C(n)$, is then normalised, such that its global minimum is offset to 0db as are all other values relative to it. The normalisation is performed by the following equation:

$$C'_{(n)} = C_{(n)} - \min\left(C_{(n)}\right)$$

The result is a set of corrective filter gains, $C'(n)$, relating to a set of frequency points, $F(n)$.

In step 2, a 2048 point linearly spaced filter kernel from the data in $C'(n)$ with respect to F(n) must be derived, so as to generate a filter kernel of correct length and which possesses the correct distribution of frequency points such as to match the parameters of the Fourier transform used to process the audio signal within the audio system, and which therefore can be used in the spectral multiplication operation of step 3.

This step requires the interpolation of the test frequency points, due to the fact that they are not inherently linearly spaced. This is achieved by first mapping the discrete test frequency points to discrete Fourier bins. The mapping function is described as follows:

$$F'_{(n)} = \frac{K \times F_{(n)}}{Fs} \qquad 1 \leq n \leq N$$

Where $K$ is the Fourier transform length of each audio frame and Fs is the sample frequency of the audio signal. Then rounding to the nearest integer, $F'(n)$ contains frequency points converted to Fourier bin indices. As $K$ points of data are needed in order carry out spectral multiplication (whereas at this point only N points of data are available), the remaining points can be calculated by interpolating the data in $C'(n)$ with respect to $F'(n)$ to a length $K$.

In one embodiment of the invention, the interpolation is performed by cubic spline interpolation. In another embodiment of the invention, the interpolation is carried out by Akima interpolation. However, it will be appreciated that any method of interpolation can be used. The interpolated data provides a compensation filter kernel, $C_{f(k)}$, of length $K$ frequency bins.

The dB values contained within $C_{f(k)}$ are then converted to multipliers, to facilitate spectral multiplication. This is performed as follows:

$$C_{f(k)} = 10^{C_f(k)/20} \qquad 0 \le k \le K$$

Where $k$ is a bin index and $K$ is the length of the Fourier Transform.

This results in a filter kernel which can be multiplied by the Fourier magnitude spectrum of each audio frame.

As mentioned above, step 3 involves applying the filter kernel to the audio by multiplying it by the instantaneous short term magnitude spectrum of the audio. This is performed by first obtaining the magnitude spectrum for the current audio frame at time $t^u$ in the signal. This can be found using the short term Fourier transform as follows:

$$X(t^u, k) = \left| \sum_{k=0}^{K-1} h(n) x(t^u + k) e^{-j\Omega_k n} \right|$$

Where x is the original signal, $h(n)$ is a windowing function (which in the described embodiment is a Hanning), and $\Omega_k = 2\pi k / N$ is the centre frequency of the $k^{th}$ bin in radians per sample, where $K$ is size of the FFT. The equation is evaluated for $0 \le k < K$ Where, $t^u$, where $u$ is the frame index. For simplification, let $X(t^u, k) = X(m,k)$ and a single frame be denoted as the $m^{th}$ frame.

This filter kernel is then applied to the audio magnitude spectrum using an elementwise multiplication, such that the newly filtered magnitude spectrum, $Y(m,k)$, is given by:

$$Y(m,k) = X(m,k) \times A \times Cf(k)$$

Where A is compensation factor allowing adjustment of the overall level of compensation. Finally, the newly filtered magnitude spectrum is then inverted back to the time domain using an inverse Fourier Transform using the original frame phases.

The present invention therefore exploits the fact that by delivering known inputs to a system, and approximately measuring the system response through user feedback, the resultant frequency response of the entire system can be derived (i.e. the system print). It should be noted that while the frequency response of the individual components in the listening chain cannot not be derived in this way, the sum response of the 'system' in its entirety may be.

It will be appreciated that in offline processing, the entire signal is overlapped and concatenated before playback. Since the buffer holding the output signal is non volatile, newly processed frames can be easily overlapped with the samples from previous iterations. However, in a real-time environment, a constant stream of processed audio must be outputted and consecutive output frames must be continuous. However, since $Y(m,k)$ is a modified complex signal, the analysis window, $h(n)$ is most certainly distorted. This implies that the filtered signal will not overlap cleanly upon resynthesis, that is, some discontinuities may be present at frame boundaries leading to clicks during playback.

In order to provide for seamless concatenation of audio frames with potentially varying levels of compensation filtering, the boundaries of each output frame must align in order to avoid distortion at the output. Since changes to the magnitude spectrum may affect the window function on inversion to the time domain, the present invention addresses this problem by enabling the output frame to be rewindowed using a 75% overlap instead of 50% in the short term Fourier transform framework. This effectively means that at any one time instant, 4 analysis frames are actively contributing to the current output frame. This could be interpreted as meaning that 4 frames of length N should be processed and overlapped before 1 frame can be output, but this is not necessarily so.

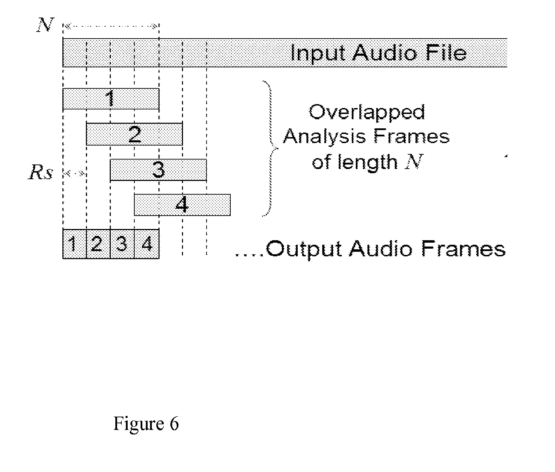

This is illustrated with respect to Figure 6, where it can be seen that the audio to be processed is divided up into overlapping frames of length N. In order to output a processed frame of length N, 4 full frames would need to be processed and overlapped. It will be appreciated that this leads to considerable latency from the time a parameter change is affected to the time when its affects are audible at the output. However, given that the synthesis hop size is fixed at Rs equal to $N/4$ (due to 75% overlapping), it is possible to in fact load and process a single frame of length N, output ¼ of it, and retain the rest in a buffer to overlap with audio in successive output frames. Here, $Rs$, known as the hopsize, is always ¼ of the frame size for a 75% overlap scheme.

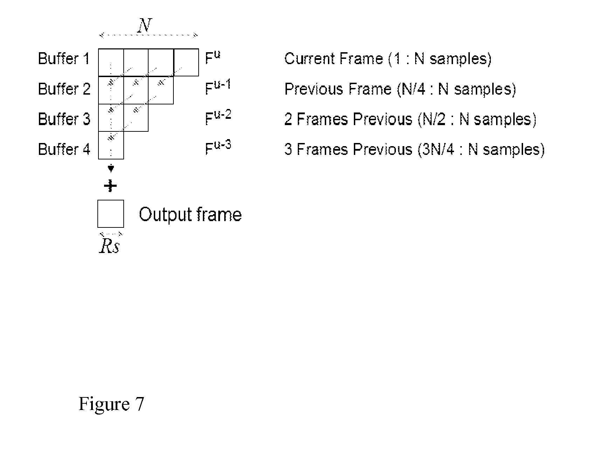

The present invention achieves this by applying the following output buffer scheme: Firstly, a buffer of length N is required in which the current processed frame (with analysis window applied) is placed. Three additional buffers of length $3N/4$, $N/2$ and $N/4$ are also required, to store remaining segments from the 3 previously processed frames. Each output frame of length $N/4$ is then generated, by summing samples from each of the 4 buffers described above. Figure 7 shows how the buffer scheme works.

Referring to Figure 6, it can be seen that on each iteration a full frame of length N is processed and placed in buffer 1. Remaining samples from the 3 previous frames occupy buffers 2, 3 and 4. The required output frame, $S^u$, of length $N/4$ is then generated as defined in the following equation:

$$S^u(n) = F^u(n) + F^{u-1}(n + \frac{N}{4}) + F^{u-2}(n + \frac{N}{2}) + F^{u-3}(n + \frac{3N}{4})$$

$$\forall n \qquad 1 \le n \le \frac{N}{4}$$

From this equation, it can be seen that the output frame is generated by summing the first $N/4$ samples form each buffer. Specifically, buffer 2 contains the remaining $3N/4$ samples from the previous frame ($F^{u-1}$). Buffer 3 contains the remaining $N/2$ samples from 2 frames previous ($F^{u-2}$), and buffer 4 contains the remaining $N/4$ samples from 3 frames previous ($F^{u-3}$). Once the output frame has been generated and outputted, the first $N/4$ samples in each buffer can be discarded. The data in all buffers must then be shifted in order to prepare for the next iteration. The arrows in Figure 7 illustrate how each segment of each buffer is shifted in order to accommodate a newly processed frame in the next iteration. The order in which the buffers are shifted is vital. Firstly, buffer 4 is filled with the remaining $N/4$ samples from buffer 3; buffer 3 is filled with the remaining $N/2$ samples from buffer 2, and finally, buffer 2 is filled with the remaining $3N/4$ samples from buffer 1. Buffer 1 is now empty and ready to receive the next processed frame of length N. The result of this scheme, is that ¼ of a processed frame will be outputted at time intervals of *Rs* which is equal to $N/4$ samples.

Where a frame size of 4096 samples is used, the output will be updated every 1024 samples, which is approximately equal to 23.2 milliseconds. The input/output latency will be larger than this, and depends on the time required to access and write to hardware buffers in the audio interface. In general however, it is possible to achieve latencies of less than 40-50ms, which is typically not discernable by the listener. This essentially allows the listener to vary the level of compensation filtering, and audition the effects on the audio in real-time. For example, in one embodiment of the invention, basic active low and high pass shelf filters can be provided which are adjustable by the listener by means of a virtual slider interface, in order to account for certain listening preferences. This is highly conducive to establishing an optimal compensation setting on the audio playback device.

In an alternative embodiment of the invention, the auditory test may be performed on both ears simultaneously. This increases the efficiency of the testing process, as it halves the time to generate the system print. This is acceptable in the context of audio reproduction devices, given that the vast majority of these devices have a single graphic equaliser, which is applied identically to both left and right audio channels. However, for users with extreme auditory imbalance, it may be preferable to perform the testing on each ear separately.

As mentioned previously, prior to commencing the auditory test and compensation process, the program to perform the process must be installed on one of the audio system components. This can be achieved in a variety of different ways. A number of these embodiments are described below.

In one embodiment, the program comprises an audio processing algorithm which has been developed for an audio device or software platform for which a third party software development environment is available. One such device is the Apple iPhone, which allows application development through the Apple iPhone SDK. The installation process will now be described with reference to the iPhone for illustrative purposes. It will be appreciated that a similar process would be performed for installation on any other similar device on which audio can be played.

In this case, the test and compensation program is developed as an iApp application for the iPhone, and must first be downloaded by the end user or listener. Once it is downloaded, the application should be installed and executed locally on the iPhone. The test is then begun, by the user launching the application, with the auditory test being delivered to the user through the headphones connected to the iPhone, as previously described with reference to Figures 2 and 3. Once the test is completed, the system print is stored to memory on the iPhone. This system print is then used to generate a listener specific system compensation profile, by the process described with reference to steps 1 and 2 of Figure 5. This profile is then stored in memory. It will be appreciated that this process needs only to be performed once. However, if any of the system components, such as for example the headphones, or the user's hearing ability change, then the entire process should be re-administered.

In order to compensate the audio being emitted from the iPhone, it is necessary to launch the compensation application on the iPhone. Within the application, the user selects their profile stored from the test process. The user can then proceed to select and listen to music as normal on the iPhone. All musical audio content will then be processed in real-time by the compensation filter generated by the application, by applying the compensation filter to the audio as previously described with reference to step 3 of Figure 5.

The application also provides the user with the option to set the level of desired compensation. This is achieved by the use of a virtual slider control on the compensation interface on the iPhone.

In another embodiment, the auditory test and compensation program is provided on a dedicated audio processing chip for inclusion in hardware. One manifestation of this includes integrating the test and compensation program into next generation headphones. The requirement for onboard processing power, memory and a power cell (battery) is implicit. In this case, an inline remote contains the control buttons to start/stop the test process, in addition to the response button for the listener to indicate sensation of the tones. The actual test is then administered locally on the headphones themselves, with the listener responding to the test in the same manner as previously described in conjunction with Figure 2, using the inline remote on the headphone lead. The system print is then captured and the resulting generated compensation filter stored to local memory. The user can activate and deactivate compensation using the inline remote. Once activated, the compensation filter processes all audio delivered to the headphones. The listener may also set the level of compensation using controls provided on the remote.

The compensation filtering may also be performed directly onto an audio file. This is known as destructive file based compensation. It will be appreciated that the compensated audio file will of course only be of value to the specific listener to whom the compensation parameters apply. In this case, the test is performed for the listener as previously described, but on a specific fit for purpose device for which the test program has been developed specifically. Once the test is complete and the user/device specific compensation profile has been generated, the compensation profile is transferred to either a local, a networked or web service, which provides access to music (for example a purchase and download service such as iTunes). This service then pre-encodes all audio tracks with the listener's compensation profile prior to download.

The present invention may also be used in the audio production environment. In this regard, it should be appreciated that audio production environments must be carefully planned and designed in order to provide the sound engineer with a faithful representation of the audio during production. The engineer must be able to make informed decisions about the sonic characteristics of the audio in order to optimise it for reproduction on a variety of consumer systems. For this reason, it is generally favourable to have a flat frequency response in terms of room acoustics and speaker response. This is generally achieved through structural and acoustic treatment within the room, and manual equalisation of speaker systems. In many cases, altering room structures is not feasible. As an alternative, some systems exist for the automatic correction of room response by using a form of compensation filtering. This requires a measurement microphone to be used to analyse chirp signals emitted within the room from the reproduction system itself. By measuring the response at the microphone, a compensation filter can be generated and applied to all audio outputs from the system for correction. However, in this instance, the listener's own hearing response is not taken into account.

The auditory test and compensation method of the present invention can be used to correct for all factors including hearing response. Furthermore, it has the added benefit that no microphone is required to measure room response. The method of the present invention in this case differs from the prior art compensation technique in that it is no longer used in conjunction with headphones, but rather on near-field speakers in an enclosed room, such as an amateur or professional recording studio. The "system" is defined as being the sound reproduction system, the actual room acoustic properties and the listener's own hearing characteristics. The sound reproduction system comprises a computer, which hosts the aforementioned audio production software to which the process of the present invention is provided by means of a plugin.

Prior to performing the test, the testing and compensation program must be installed on a computer with a compatible audio production host application, which is connected to a sound reproduction system including a near-field speaker system. In this case, two applications must be installed -namely the testing application and the compensation plugin. The compensation plugin is registered by the host application. The compensation plugin is developed by the use of a software development kit (SDK), which is typically supplied by the manufacturers of professional audio production systems for third party developers.

Once both applications are installed, the user can launch the test application. The required reference level (listening volume) must be set first. Specifically, the user will increase the system volume until a minimal audible reference tone can be heard. After this, the auditory test continues as previously described, with the tones being output through the reproduction system via the near-field speakers, and the listener responding to sensation by depressing either the mouse or keyboard. The system print is then captured and the compensation profile generated and stored to local memory, in the same manner as previously described. This should provide compensation for aberrations in the room, the speaker and the listener response.

In order to use the compensation profile during an audio mix or production session, the user must open the host application. The user then navigates to the insert panel of the master bus, which is where all audio tracks are accumulated prior to output. The insert menu should display all of registered plugins, one of which should be the compensation application. Once this is selected, it is now automatically placed in the audio processing chain of the host application. The user should then enter the compensation plugin control panel and select their compensation profile from the menu of captured profiles. The user also has the ability to set the level of compensation to be applied from this control panel.

Once these steps are carried out, a user can perform audio production tasks such as mixing and mastering with the compensation plugin switched on, so as to apply compensation on the audio in accordance with the generated compensation profile. When the session is complete, the compensation should be switched off, prior to rendering the final mix. This is due to the fact that the compensation is room and listener specific. However, because the engineer has had the benefit of optimal listening conditions, optimal mixes which should translate to a wide range of reproduction systems can be created.

The present invention provides for the improvement in the listening experience for a user so as to provide an optimal listening experience from a particular audio device to the user, or during audio production. Furthermore, the invention supports real-time application and manipulation of filter parameters such that the end user can fine tune aspects of the compensation filter and also alter basic filter parameters so as to accommodate different listening scenarios. In addition, it should be appreciated that unlike existing audiometric techniques, this methodology allows for any subset of frequency points between 20 Hz and 20 KHz to be used.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

**Claims**

1. An auditory test method for an audio system comprising an audio device coupled to an audio output means and a listener of the audio device, the method comprising the steps of:

   delivering a series of audio stimuli through the audio output means;
   capturing a listener's response to the audibility of the stimuli; and
   generating a frequency response for the audio system based on the captured data.

2. The method of Claim 1, wherein the method further comprises the steps of:

   a) generating a tone of a predefined frequency and amplitude;
   b) repeatedly reducing the amplitude of the generated tone until the captured response indicates an amplitude value which is not audible to the listener;
   c) storing the relative amplitude value as the threshold amplitude value of the listener for the frequency;
   d) repeating steps a) to c) for a plurality of different frequencies within a predefined frequency range; and
   e) generating the frequency response for the audio system from the stored threshold amplitude values for the frequencies.

3. The method of Claim 2, further comprising the steps of:

   generating a band limited noise burst encapsulating a plurality of frequencies greater than the predefined frequency range;
   determining from the captured response the threshold frequency value of the listener for the band limited noise burst; and
   generating the frequency response for the audio system based on the stored threshold amplitude values and the threshold frequency value of the band limited noise burst.

4. The method of Claim 3, wherein the predefined frequency range is a range between 20Hz and 15Khz, and the band limited noise burst encapsulates frequencies between 15KHz and 20kHz.

5. The method of any of Claims 2 to 4, wherein the frequencies are derived from the closest critical band centres to the test frequencies utilised in the ISO 226 standard.

6. The method of any of Claims 2 to 5, further comprising the steps of:

generating a first chirp signal containing the threshold amplitude values for the frequencies;
determining from the captured response whether the chirp signal is audible to the listener;
generating a second chirp signal containing amplitude values less than the threshold amplitude values for the frequencies;
determining from the captured response whether the second chirp signal is audible to the listener; and
indicating that the generated frequency response for the audio system is correct if all of the frequencies in the first chirp signal are audible and the second chirp signal is not audible to the listener.

7. A compensation method for an audio system associated with a frequency response which has been previously generated by the performance of an auditory test on one or more of the system components, the system comprising an audio device coupled to an audio output means and a listener of the audio device; the method comprising:

d) calculating a compensation print from the frequency response;
e) deriving a filter from the calculated compensation print with respect to the frequencies associated with the frequency response;
f) applying the filter to an audio signal of the audio system.

8. The method of Claim 7, wherein the step of calculating the compensation print comprises:

performing a filter transformation which maps the frequency response to the ideal system response; and normalising the resultant vector.

9. The method of Claim 7, wherein the step of deriving the filter further comprises the steps of:

mapping the frequency points associated with the frequency response to discrete fourier bins; and interpolating the calculated compensation print values with respect to the fourier bins indices so as to provide a compensation filter kernel having a length corresponding to the fourier transform length of each audio frame.

10. The method of Claim 7, wherein the step of applying the filter to the audio signal comprises the step of:

multiplying the filter kernel by the instantaneous short term fourier magnitude spectrum of each audio frame.

11. An auditory test and compensation method for an audio system comprising an audio device coupled to an audio output means and a listener of the audio device comprising:

generating a frequency response for the audio system in accordance with the steps of the auditory test method of any of Claims 1 to 6; and
compensating for the frequency response of the audio system in accordance with the steps of the compensation method of any of Claims 7 to 10.

12. An audio system comprising:

an audio device; and
an audio output means coupled to the audio device; and
a capturing means for capturing a listener's response to a series of audio stimuli delivered through the audio output means; and
a processor adapted to perform the auditory test and compensation method of Claim 11.

13. The audio system of Claim 12 wherein the processor is provided in the audio device or in the audio output means.

14. A system comprising:

an audio system comprising an audio device coupled to an audio output means; and
a remote source for storing audio content for downloading to the audio device;
wherein the audio device is adapted to perform the auditory test method of any of Claims 1 to 6 and upload the generated frequency response to the remote source; and the remote source is adapted to perform the compensation method of any of Claims 7 to 10 on the frequency response and download the filtered audio signal to the audio device.

**15.** An in-ear device programmed with a frequency response generated in accordance with the steps of the auditory test method of any of Claims 1 to 6 and adapted to compensate for the frequency response in accordance with the steps of the compensation method of any of Claims 7 to 10.

Audio device

Audio output
means

Listener

Figure 1

Figure 2

Deliver a tone of known frequency and known relative amplitude to the ear of a listener and capture the listener's response

↓

Repeatedly reduce the amplitude of the tone until a threshold amplitude is reached when the captured data indicates the listener has no sensation and store this value

↓

Repeat steps 1 and 2 for a plurality of frequencies in a predetermined frequency range

↓

Repeat steps 1 to 3 for the other ear

↓

Generate a frequency response for the system from the captured data

↓

Determine the accuracy of the frequency response

Figure 3

3a — Sequentially test a plurality of predetermined optimum frequencies

3b — Band limit noise test higher frequencies

Figure 4

| 1 | Calculate a normalised compensation print from the system print |
|---|---|

| 2 | Derive a filter kernel from the normalised compensation print |
|---|---|

| 3 | Apply the compensation to the audio system by multiplying the short-term magnitude spectrum of the audio content of the audio device by the compensation filter kernel |
|---|---|

Figure 5

Figure 6

Figure 7

Europäisches
Patentamt

European
Patent Office

Office européen
des brevets

# EUROPEAN SEARCH REPORT

Application Number

EP 09 16 9425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 21 01 553 A1 (PHONAK GES SAPPER & CO DEUTSCH) 27 July 1972 (1972-07-27) | 1,2 | INV. A61B5/12 H01R25/00 |
| A | * pages 5-7 * | 3-6, 11-14 | |
| A | US 2 511 482 A (SHAPER HARRY B) 13 June 1950 (1950-06-13) * column 1, last paragraph - column 2, paragraph 1 * | 3 | |
| A | US 2003/101215 A1 (PURIA SUNIL [US] ET AL) 29 May 2003 (2003-05-29) * paragraphs [0019], [0023] * | 3 | |
| A,D | WO 2007/112918 A1 (CLEARTONE TECHNOLOGIES LTD [IE]; GANTER DECLAN B [IE]; GRAHAM FINTAN J) 11 October 2007 (2007-10-11) * the whole document * & EP 2 005 792 A1 (CLEARTONE TECHNOLOGIES LTD [IE]) 24 December 2008 (2008-12-24) | 1-6, 11-14 | |
| X | WO 03/026349 A1 (SOUND ID [US]; RADAR SCOTT R [US]; MENZEL CHRISTOPH [US]; EDWARDS BREN) 27 March 2003 (2003-03-27) * paragraphs [0007], [0013], [0038], [0046], [0068]; figures 1,2 * | 1,7-15 | TECHNICAL FIELDS SEARCHED (IPC) A61B H01R |
| X | US 2002/068986 A1 (MOULINE ALI [US]) 6 June 2002 (2002-06-06) * paragraphs [0025] - [0028], [0039] - [0042]; figure 3 * | 1,7-15 | |
| X | WO 2008/003525 A1 (SONY ERICSSON MOBILE COMM AB [SE]; HANSSON MAGNUS [CA]) 10 January 2008 (2008-01-10) * page 8, line 15 - page 10, line 2 * | 1,7-15 | |

-/--

~~The present search report has been drawn up for all claims~~

| Place of search | Date of completion of the search | Examiner |
|---|---|---|
| Berlin | 17 September 2010 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 09 16 9425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 7 042 986 B1 (LASHLEY DAVID GLENN [US] ET AL) 9 May 2006 (2006-05-09) * abstract; figures 8-10 * ----- | 1,7-15 | |
| | | | **TECHNICAL FIELDS SEARCHED (IPC)** |

The present search report has been drawn up for all claims

| Place of search | Date of completion of the search | Examiner |
|---|---|---|
| Berlin | 17 September 2010 | Clevorn, Jens |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

....................................................................................................
& : member of the same patent family, corresponding
   document

EPO FORM 1503 03.82 (P04C01)

Europäisches
Patentamt

European
Patent Office

Office européen
des brevets

**Application Number**

EP 09 16 9425

| CLAIMS INCURRING FEES |
|---|

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

| LACK OF UNITY OF INVENTION |
|---|

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-4, 7-10, 15(completely); 5, 6, 11-14(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

**Application Number**

EP 09 16 9425

The Search Division considers that the present European patent application does not comply with the requirements of unity of invention and relates to several inventions or groups of inventions, namely:

1. claims: 1-4(completely); 5, 6, 11-14(partially)

   Generating a band limited noise burst encapsulating a plurality of frequencies greater than the predefined frequency range; determining from the captured response the threshold frequency value of the listener for the band limited noise burst; and generating the frequency response for the audio system based on the stored threshold amplitude values and the threshold frequency value of the band limited noise burst

   ---

2. claims: 5, 6(completely); 11-14(partially)

   Frequencies are derived from the closest critical band centres to the test frequencies utilised in the ISO 226 standard

   ---

3. claims: 7-10, 15(completely); 11-14(partially)

   Compensation method for an audio system associated with a frequency response which has been previously generated by the performance of an auditory test on one or more of the system components, the system comprising an audio device coupled to an audio output means and a listener of the audio device; the method comprising: d) calculating a compensation print from the frequency response; e) deriving a filter from the calculated compensation print with respect to the frequencies associated with the frequency response; f) applying the filter to an audio signal of the audio system

   ---

**ANNEX TO THE EUROPEAN SEARCH REPORT
ON EUROPEAN PATENT APPLICATION NO.**

EP 09 16 9425

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2010

| Patent document cited in search report | | Publication date | Patent family member(s) | | Publication date |
|---|---|---|---|---|---|
| DE 2101553 | A1 | 27-07-1972 | NONE | | |
| US 2511482 | A | 13-06-1950 | NONE | | |
| US 2003101215 | A1 | 29-05-2003 | NONE | | |
| WO 2007112918 | A1 | 11-10-2007 | CA 2653767 A1 | | 11-10-2007 |
| | | | EP 2005792 A1 | | 24-12-2008 |
| | | | JP 2009532148 T | | 10-09-2009 |
| | | | US 2010191143 A1 | | 29-07-2010 |
| WO 03026349 | A1 | 27-03-2003 | CN 1589588 A | | 02-03-2005 |
| | | | EP 1438874 A1 | | 21-07-2004 |
| | | | JP 2005504470 T | | 10-02-2005 |
| | | | US 2005260978 A1 | | 24-11-2005 |
| | | | US 2005260985 A1 | | 24-11-2005 |
| | | | US 2003064746 A1 | | 03-04-2003 |
| US 2002068986 | A1 | 06-06-2002 | NONE | | |
| WO 2008003525 | A1 | 10-01-2008 | AT 463924 T | | 15-04-2010 |
| | | | CN 101467431 A | | 24-06-2009 |
| | | | EP 2039135 A1 | | 25-03-2009 |
| | | | US 2008008328 A1 | | 10-01-2008 |
| US 7042986 | B1 | 09-05-2006 | NONE | | |

EPO FORM P0459

For more details about this annex : see Official Journal of the European Patent Office, No. 12/82

REFERENCES CITED IN THE DESCRIPTION

**Patent documents cited in the description**

- EP 2005792 A